# DEMANDE DE BREVET EUROPEEN

(11) **EP 1 247 504 A1**
(43) Date de publication de la demande: **09.10.2002**
(21) Numéro de dépôt: 02356067.5
(22) Date de dépôt: 05.04.2002
(51) Int. Cl.: A61F 5/14, A43D 39/00, A43D 1/02, B29C 43/02

(54) **Procédé et dispositif de fabrication d'orthèses plantaires**

(30) Priorité: 05.04.2001 FR 0104653
(71) Demandeur: SOCIETE D'IMPORTATION DE DIFFUSION OU DISTRIBUTION D'ARTICLES DE SPORT-S.I.D.A.S., 38500 Voiron (FR)
(72) Inventeur: Polidoro, Jean-Pierre, 26380 Peyrins (FR)
(74) Mandataire: Guerre, Dominique

(57) **Abrégé**

Ce procédé consiste à déterminer les caractéristiques anatomiques de la face inférieure d'un pied, à choisir une forme (23) ou à réaliser un moulage ayant la forme de la face inférieure de ce pied, à définir l'épaisseur de l'orthèse à réaliser, à placer dans un moule (14) comportant un fond (15) galbé au profil de la première de montage de la chaussure à laquelle est destinée l'orthèse, et une paroi latérale (16), au moins deux couches (3) de matière synthétique à l'état malléable, à réaliser le positionnement de la forme (23) ou du moulage du pied sur son support (9) en tenant compte des caractéristiques anatomiques de la face inférieure du pied, et à réaliser le déplacement relatif de la forme (23) et du moule (14) pour comprimer la matière synthétique et mettre en forme celle-ci, tout en réalisant l'assemblage des différentes couches.

## Description

La présente invention a pour objet un procédé et un dispositif de fabrication d'orthèses plantaires.

Une orthèse plantaire est destinée à équiper une chaussure, pour réaliser l'interface entre cette chaussure et le pied d'une personne présentant un état pathologique, ou des caractéristiques anatomiques nécessitant une correction.

De façon connue, une orthèse plantaire est constituée par superposition de plusieurs couches de matériaux, qui sont assemblées les unes aux autres, et qui sont mises en forme par ponçage au fur et à mesure de l'assemblage. Il faut tenir compte des formes respectives de la face inférieure de l'orthèse, qui correspond au profil de la première de montage de la chaussure, de la face supérieure de l'orthèse qui est adaptée à la forme du pied à équiper, mais aussi des caractéristiques anatomiques de ce pied, pour tenir compte d'une orientation en varus-valgus, et d'une orientation equin-talus.

La technique la plus ancienne consiste à utiliser des plaques de liège.

Plus récemment ont été utilisées des plaques en mousse thermoplastique, qui sont collées ou soudées les unes sur les autres, lors de leur superposition.

Il résulte de cette technique qu'elle doit être mise en oeuvre par un spécialiste, que le temps d'exécution d'une orthèse est important, ce qui implique un coût de réalisation élevé et que le résultat dépend de la compétence de l'opérateur.

Le but de l'invention est de fournir un procédé et un dispositif permettant d'industrialiser la fabrication d'orthèses plantaires en obtenant une excellente qualité et une constance de fabrication des orthèses, tout en réduisant le prix de revient de celles-ci.

A cet effet, le procédé qu'elle concerne consiste :
- à déterminer les caractéristiques anatomiques de la face inférieure d'un pied d'une personne,
- à choisir une forme ou à réaliser un moulage ayant la forme de la face inférieure de ce pied,
- à définir l'épaisseur de l'orthèse à réaliser, en fonction de l'anatomie de la personne à équiper,
- à placer dans un moule comportant un fond galbé au profil de la première de montage de la chaussure à laquelle est destinée l'orthèse, et une paroi latérale, au moins deux couches de matière synthétique thermoplastique ou thermodurcissable, l'épaisseur cumulée des couches étant supérieure à l'épaisseur de l'orthèse à réaliser, cette matière synthétique étant préalablement amenée à un état malléable,
- à réaliser le positionnement de la forme ou du moulage du pied sur son support en tenant compte des caractéristiques anatomiques de la face inférieure du pied,
- et à réaliser le déplacement relatif de la forme et du moule pour comprimer la matière synthétique et mettre en forme celle-ci, tout en réalisant l'assemblage des différentes couches, en tenant compte, dans le positionnement final de la forme et du moule, de l'épaisseur souhaitée pour l'orthèse.

Suivant un premier mode de mise en oeuvre ce procédé consiste, à définir le nombre de plaques constituant les couches de matière thermoplastique ou thermodurcissable en fonction de l'épaisseur définitive de l'orthèse et des caractéristiques des plaques, telles que dureté de celles-ci, à réaliser la découpe de chaque plaque au profil de la forme avec ménagement d'un débord, à réaliser le chauffage individuel des plaques pour les amener à un état malléable, et à les superposer dans le moule avant de procéder à l'opération de mise en forme et d'assemblage par compression.

Avantageusement, l'épaisseur cumulée des différentes plaques, en position non comprimée, est supérieure d'au moins 20% à l'épaisseur de l'orthèse à réaliser.

Enfin, ce procédé consiste, après moulage et refroidissement de la matière synthétique, à supprimer la partie débordant de la forme pour obtenir l'orthèse. Il s'agit de la seule phase de ce procédé qui n'est pas automatisée. La suppression de la partie débordante peut être réalisée par meulage.

La mise en forme de la face supérieure et de la face inférieure de l'orthèse sont réalisées de façon très précise, grâce aux formes, respectivement du fond du moule et de la forme ou du moulage du pied, l'épaisseur de l'orthèse étant réglée par détermination de la distance entre la face inférieure du moule et la face inférieure de la forme ou du moulage du pied, en position de moulage.

Un dispositif de fabrication d'orthèse comprend un bâti sur lequel est destiné à être fixé, de façon réglable, une forme ou un moulage d'un pied et un plateau qui, portant un moule destiné à recevoir la matière synthétique constitutive de l'orthèse, est monté sur des moyens assurant son déplacement pour réaliser l'engagement de la forme dans le moule.

Suivant une caractéristique de ce dispositif, le bâti comprend une poutre horizontale traversée par une colonne verticale et réglable verticalement dont l'extrémité inférieure est équipée de moyens de fixation de l'extrémité supérieure d'une forme ou d'un moulage de pied, le moule étant fixé sur un plateau horizontal monté à l'extrémité supérieure d'une colonne verticale déplaçable sous l'action d'un vérin d'axe vertical, tel qu'un vérin pneumatique.

La colonne verticale peut être réglée verticalement par blocage à l'aide d'un dispositif de serrage, par exemple à excentrique, ou comporter une partie vissée, engagée dans un écrou solidaire de la poutre horizontale.

Il est en effet important de positionner de façon précise la forme, car c'est de la position de la forme que dépendra l'épaisseur de l'orthèse, dans la mesure où la course du vérin est prédéterminée.

Avantageusement, l'extrémité inférieure de la colonne servant à la fixation de la forme comporte deux axes d'articulation horizontaux croisés et superposés, un axe orienté longitudinalement à la forme et un axe transversal à celle-ci, avec possibilité de blocage dans la position angulaire souhaitée.

Cette caractéristique permet de régler la position de la forme en tenant compte d'éventuelles déformations ou positionnements du pied suivant des inclinaisons valgus-varus et equin-talus.

Afin d'assurer une bonne tenue de la forme qui n'est fixée que par sa partie supérieure, au cours de la phase de compression, ce dispositif comprend une seconde colonne verticale parallèle à la colonne portant la forme, réglable verticalement et équipée, à son extrémité inférieure, d'une pièce d'appui sur la face supérieure de la forme, située en regard de la zone métatarsienne.

Afin de permettre un réglage précis du positionnement de la forme, la poutre traversée par chaque colonne de fixation ou de maintien d'une forme est équipée de réglettes graduées indicatrices de la position de chaque colonne et par suite de la position de la forme par rapport au bâti.

Suivant une autre caractéristique de l'invention, le moule est monté pivotant sur le plateau support, avec possibilité de blocage dans la position de moulage. Ce pivotement permet de dégager le moule par rapport à la forme, pour réaliser la mise en place des plaques de matériau thermoplastique devant composer l'orthèse, et pour permettre le retrait du produit moulé, après moulage par compression.

De toute façon, l'invention sera bien comprise à l'aide de la description qui suit, en référence au dessin schématique annexé représentant, à titres d'exemples non limitatifs, une orthèse et un dispositif pour la fabrication de celle-ci.
Figure 1 est une vue en perspective d'une orthèse,
Figure 2 est une vue en perspective d'un dispositif pour la fabrication de cette orthèse,
Figures 3 et 4 sont deux vues de côté de ce dispositif respectivement avant l'opération du moulage et pendant l'opération de moulage,
Figure 5 est une vue en perspective et à échelle agrandie représentant les moyens de réglage de la position de la forme, avant moulage.

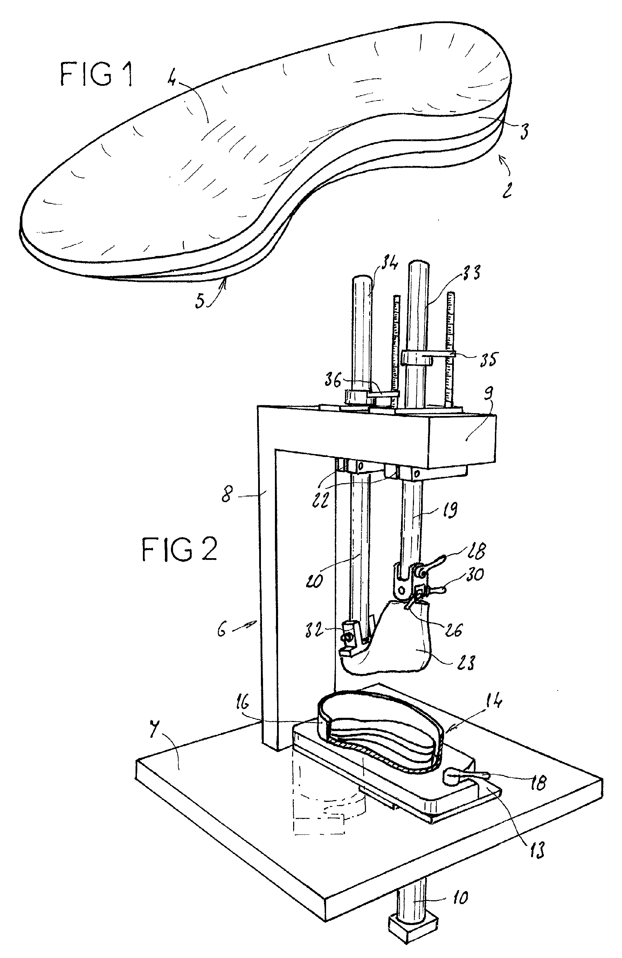

La figure 1 représente une orthèse 2 constituée par trois plaques 3 de matière thermoplastique. Cette forme 3 comprend une face supérieure 4 adaptée à l'anatomie du pied d'un individu, et une face inférieure 5 adaptée à la forme de la première d'une chaussure dans laquelle est destinée à être montée l'orthèse.

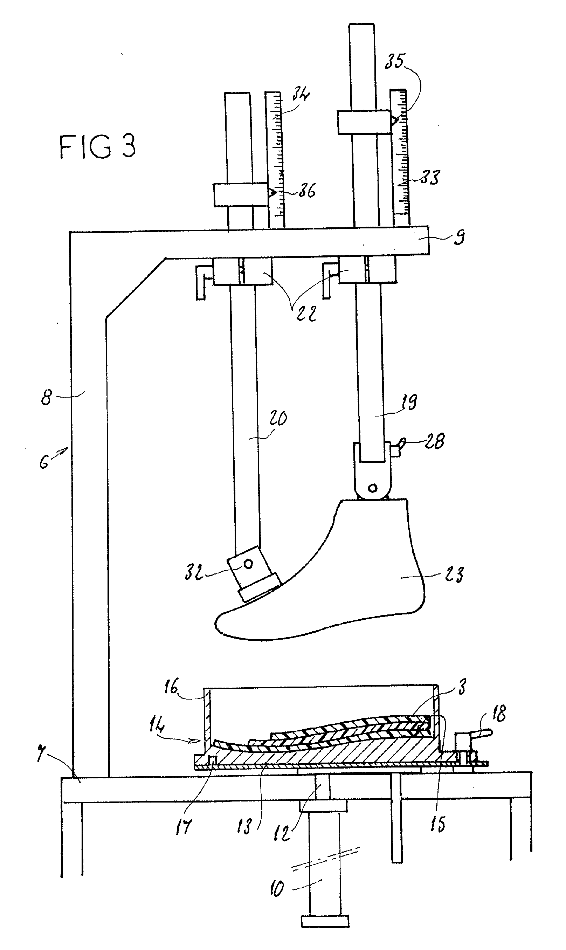

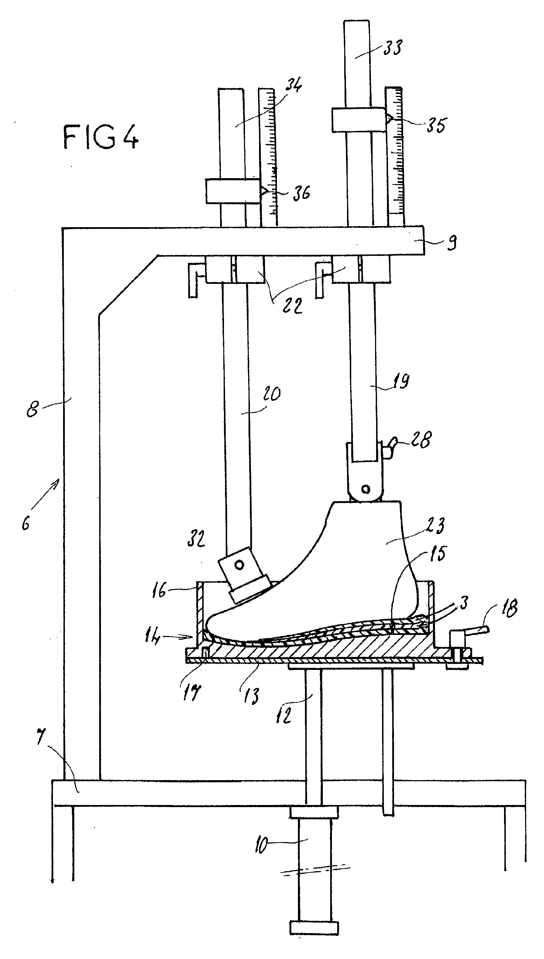

Le dispositif pour la réalisation de cette orthèse représenté aux figures 2 à 5 comprend un bâti 6 présentant un support 7, sur la face supérieure duquel est fixée une poutre verticale 8, à partir de laquelle s'étend une poutre horizontale 9 formant potence. Sous le plateau 7 est disposé un vérin pneumatique 10 comportant une tige 12 à l'extrémité de laquelle est fixé un plateau support 13. Le vérin pneumatique est susceptible de développer une pression supérieure à 10 bars. Sur le plateau support 13 est monté un bloc moule 14, le moule 14 comportant un fond 15 galbé de façon correspondant à la première de montage d'une chaussure, ce fond étant bordé par une paroi latérale 16. Pour des raisons de commodité pour l'utilisateur, le bloc moule 14 est monté pivotant sur le plateau 13 autour d'un axe vertical 17, avec possibilité de verrouillage par un levier 18, dans la position de moulage. La course du vérin entre une position basse représentée à la figure 3 et une position haute représentée à la figure 4 est prédéterminée.

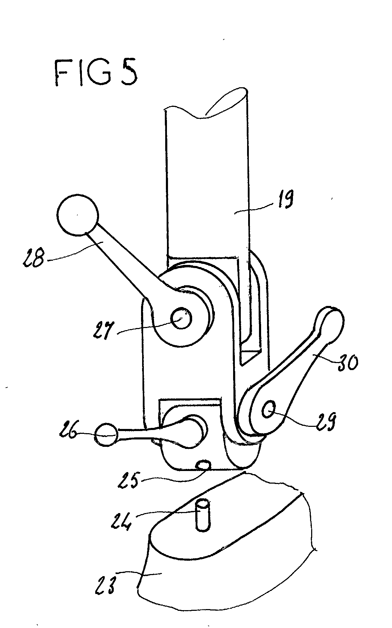

Ce dispositif comprend également deux colonnes 19 et 20, verticales, traversant la poutre horizontale 9, et pouvant l'une et l'autre être bloquées vis à vis de cette poutre par deux dispositifs adaptés, tel que des dispositifs à excentriques 22. L'extrémité inférieure de la colonne 19 est équipée de moyens de fixation d'une forme 23 ou du moulage d'un pied. Le dispositif de fixation de la forme 23 est plus spécialement représenté à la figure 5. La forme 23 comporte à son extrémité supérieure une tige 24 engagée dans un orifice 25 du dispositif de fixation, avec blocage à l'aide d'un levier 26. Le dispositif de fixation de la forme sur la colonne 19 est réalisé avec deux articulations croisées, une articulation autour d'un axe 27 pour permettre une orientation de la forme 23 pour des corrections valgus-varus, avec blocage dans la position souhaitée par un levier 28, et une inclinaison de la forme autour d'un axe 29 avec blocage par un levier 30, pour tenir compte de spécificités de positionnement dans le sens equin-talus.

La colonne 20 comporte à son extrémité inférieure une pièce d'appui 32 sur la face supérieure de la forme, en regard de la zone métatarsienne. Des réglettes graduées 33 et 34 sont associées aux deux colonnes 19 et 20 qui comportent pour leur part des repères 35 et 36 en vis à vis de ces réglettes.

D'un point de vue pratique, le moule 14 se trouvant en position dégagée, comme représenté en traits mixtes à la figure 2, vis à vis du plateau support 13, le praticien dépose dans le moule plusieurs plaques 3 de matière thermoplastique, dont la surface est telle qu'elle déborde par rapport à la surface de la forme, et que l'épaisseur totale est légèrement supérieure, d'au moins 20%, par exemple de l'ordre de 1 à 3 cm à l'épaisseur désirée de l'orthèse.

Après un pré-positionnement des plaques de matière thermoplastique, pour vérifier leur conformité avec le fond du moule, les plaques sont placées dans un four où elles sont chauffées pour être amenées à un état malléable.

La forme est positionnée dans le sens vertical, ainsi que longitudinalement et transversalement, pour tenir compte de l'épaisseur de semelle désirée, c'est-à-dire de la distance entre le fond du moule et la forme, en position de compression, après avoir tenu compte des inclinaisons longitudinale et transversale de la forme adaptée à l'anatomie du pied. La colonne 20 prenant appui par la pièce 32 sur la partie supérieure de la forme, évite que celle-ci ne subisse une déformation vis à vis de son point de fixation 24, 25 lors de la phase de compression. Pour permettre le réglage vertical de la forme, les réglettes 33 et 34 et les repères associés 35 et 36 des deux colonnes 19 et 20 sont mis à profit.

Une fois les plaques en matière thermoplastique 3 amenées à un état malléable, elles sont positionnées dans le fond du moule, comme montré à la figure 3, après quoi le moule est déplacé vers le haut par action du vérin 10, mouvement au cours duquel les plaques de matière thermoplastique viennent en contact avec la face inférieure de la forme 23 et épouse cette dernière. Le maintien en compression est réalisé tant que la matière n'a pas refroidi. Cette opération permet la mise en forme des plaques et leur assemblage, par compression.

Après refroidissement, le moule est abaissé vers la position représentée à la figure 3, et il est procédé à son pivotement pour retirer une ébauche d'orthèse qui est ensuite terminée par une opération de meulage de sa périphérie pour lui donner la forme de la première de montage de la chaussure.

Il doit être noté que pour faciliter le démoulage de l'ébauche d'orthèse, le bord délimitant la paroi latérale du moule est légèrement évasée.

Comme il ressort de ce qui précède l'invention apporte une grande amélioration à la technique existante en fournissant un procédé et un dispositif permettant de réaliser une orthèse au cours d'une seule opération par moulage par compression. Ce procédé est simple et rapide de mise en oeuvre, et parfaitement reproductible.

Comme il va de soi l'invention ne se limite pas à la seule forme d'exécution de ce dispositif ni à la seule forme d'exécution de cette orthèse décrits ci-dessus à titre d'exemples. Elle en embrasse au contraire toutes les variantes. C'est ainsi notamment que le déplacement des colonnes du dispositif pourrait être réalisé différemment, par exemple à l'aide d'une colonne à vis, ou d'un dispositif automatisé, sans que l'on sorte pour autant du cadre de l'invention.

## Revendications

1. Procédé de fabrication d'orthèses plantaires **caractérisé en ce qu'**il consiste :
- à déterminer les caractéristiques anatomiques de la face inférieure d'un pied d'une personne,
- à choisir une forme (23) ou à réaliser un moulage ayant la forme de la face inférieure de ce pied,
- à définir l'épaisseur de l'orthèse à réaliser, en fonction de l'anatomie de la personne à équiper,
- à placer dans un moule (14) comportant un fond galbé au profil de la première de montage de la chaussure à laquelle est destinée l'orthèse, et une paroi latérale (16), au moins deux couches (3) de matière synthétique thermoplastique ou thermodurcissable, l'épaisseur cumulée des couches étant supérieure à l'épaisseur de l'orthèse à réaliser, cette matière synthétique étant préalablement amenée à un état malléable,
- à réaliser le positionnement de la forme (23) ou du moulage du pied sur son support (9) en tenant compte des caractéristiques anatomiques de la face inférieure du pied,
- et à réaliser le déplacement relatif de la forme (23) et du moule (14) pour comprimer la matière synthétique et mettre en forme celle-ci, tout en réalisant l'assemblage des différentes couches, en tenant compte, dans le positionnement final de la forme et du moule, de l'épaisseur souhaitée pour l'orthèse.

2. Procédé selon la revendication 1, **caractérisé en ce qu'**il consiste à définir le nombre de plaques constituant les couches de matière thermoplastique ou thermodurcissable en fonction de l'épaisseur définitive de l'orthèse et des caractéristiques des plaques, telles que dureté de celles-ci, à réaliser la découpe de chaque plaque (3) au profil de la forme (23) avec ménagement d'un débord, à réaliser le chauffage individuel des plaques pour les amener à un état malléable, et à les superposer dans le moule (14) avant de procéder à l'opération de mise en forme et d'assemblage par compression.

3. Procédé selon l'une des revendications 1 et 2, **caractérisé en ce que** l'épaisseur cumulée des différentes plaques (3), en position non comprimée, est supérieure d'au moins 20% à l'épaisseur de l'orthèse à réaliser.

4. Procédé selon l'une des revendications 1 à 3, **caractérisé en ce qu'**il consiste, après moulage et refroidissement de la matière synthétique, à supprimer la partie débordant de la forme pour obtenir l'orthèse.

5. Dispositif pour la fabrication d'orthèses plantaires, **caractérisé en ce qu'**il comprend un bâti (6) sur lequel est destiné à être fixé, de façon réglable, une forme (23) ou un moulage d'un pied et un plateau (13) qui, portant un moule (14) destiné à recevoir la matière synthétique constitutive de l'orthèse, est monté sur des moyens (10, 12) assurant son déplacement pour réaliser l'engagement de la forme dans le moule.

6. Dispositif selon la revendication 5, **caractérisé en ce que** le bâti comprend une poutre horizontale (9) traversée par une colonne (19) verticale et réglable verticalement dont l'extrémité inférieure est équipée de moyens de fixation (24, 25) de l'extrémité supérieure d'une forme (23) ou d'un moulage de pied, le moule (14) étant fixé sur un plateau horizontal (13) monté à l'extrémité supérieure d'une colonne verticale (12) déplaçable sous l'action d'un vérin (10) d'axe vertical, tel qu'un vérin pneumatique.

7. Dispositif selon la revendication 6, **caractérisé en ce que** la course du vérin (10) est prédéterminée.

8. Dispositif selon l'une des revendications 6 et 7, **caractérisé en ce que** l'extrémité inférieure de la colonne (19) servant à la fixation de la forme (23) comporte deux axes d'articulation horizontaux (27, 29) croisés et superposés, un axe orienté longitudinalement à la forme et un axe transversal à celle-ci, avec possibilité de blocage dans la position angulaire souhaitée.

9. Dispositif selon l'une des revendications 6 à 8, **caractérisé en ce qu'**il comprend une seconde colonne verticale (20) parallèle à la colonne (19) portant la forme, et réglable verticalement et équipée, à son extrémité inférieure, d'une pièce d'appui (32) sur la face supérieure de la forme (23), située en regard de la zone métatarsienne.

10. Dispositif selon l'une des revendications 6 et 9, **caractérisé en ce que** la poutre (9) traversée par chaque colonne (19, 20) de fixation ou de maintien d'une forme est équipée de réglettes graduées (33, 34) indicatrices de la position de chaque colonne (19, 20) et par suite de la position de la forme (23) par rapport au bâti.
